# EUROPEAN PATENT APPLICATION

(11) **EP 2 180 015 A1**
(43) Date of publication of application: **28.04.2010**
(21) Application number: 08805343.4
(22) Date of filing: 23.07.2008
(51) Int. Cl.: C08G 73/10, C07C 217/90, H01L 41/193

(54) **POLYIMIDES WITH PIEZOELECTRIC PROPERTIES**

(30) Priority: 23.07.2007 ES 200702046
(71) Applicant: Fundacion Gaiker, 48170 Zamudio Vizcaya (ES); Universidad Del Pais Vasco - Euskal Herriko Unibertsitatea, 48940 Leioa Vizcaya (ES)
(72) Inventor: GONZALO GONZALEZ, Beatriz, E-48170 Zamudio (Vizcaya) (ES); DIOS PEON, José, Ramón, E-48170 Zamudio (Vizcaya) (ES); VILAS VILELA, José, Luis, E-48940 Leioa (Vizcaya) (ES); LEON ISIDRO, Luis, Manuel, E-48940 Leioa (vizcaya) (ES); BRECZEWSKI, Tomasz, E-48940 Leioa (Vizcaya) (ES); PEREZ JUBINDO, Miguel, Angel, E-48940 Leioa (Vizcaya) (ES); DE LA FUENTE LAVIN, Maria, Rosario, E-48940 Leioa (Vizcaya) (ES)
(74) Representative: Carpintero Lopez, Francisco
(86) International application number: PCT/ES2008/000514
(87) International publication number: WO 2009/013376

(57) **Abstract**

The invention defines polyimides with good piezoelectric properties at high temperatures having a repeating structural unit of formula (I) wherein X and Y represent hydrogen and cyano, with the proviso that when one of X and Y is hydrogen, the other one is cyano. The invention also defines a process for the preparation of these polyimides. It likewise defines diamines for the preparation thereof and a process for obtaining these diamines. Finally, the invention defines the use of said polyimides in a wide range of industrial applications since they have good remanent polarization values as well as stability at high temperatures and are furthermore flexible and lightweight.

## Description

### Field of the Invention

The invention relates to the field of piezoelectric materials. The invention particularly relates to novel polyimides having good piezoelectric properties at high temperatures and which can therefore be used in applications in which piezoelectricity is required at temperatures greater than 90ºC, flexibility and low weight of the piezoelectric device.

### Background of the Invention

As is known in the state of the art, piezoelectric materials are broadly used due to their fast electromagnetic response and their relatively low energy requirements. A classic definition of piezoelectricity, a Greek term for electricity under pressure, is the generation of electric polarization of a material in response to mechanical stress. This phenomenon is known as direct effect or generator effect and is essentially used for sensors. Piezoelectric materials also experience the inverse effect or motor effect, i.e., mechanical deformation under the application of an electric charge or signal, and are essentially used for actuators.

Piezoelectricity is a linear effect relating to the structure of the solid. The microscopic origin of the piezoelectric effect is the displacement of electric charges within the structure of the solid.

Rochelle salt, obtained in France in 1665 by pharmacist Elie Seignette for applications in medicine, is the first known piezoelectric material (Busch G., Early History of Ferroelectricity. Ferroelectrics, 74, 267, (1987); and Kanzig, W., History of Ferroelectricity. Ferroelectrics, 74, 285, (1987)). Research on piezoelectric materials continued at the beginning of the 20^{th} century driven, in part, by military interests in World War I. Piezoelectric quartz was used in acoustic projectors and in earphones. Interest in the study of piezoelectric materials subsequently grew in World War II for radio communications and for underwater acoustic applications.

Piezoelectric ceramics, such as lead zirconium titanate (PZT) ceramics, have been used in the last four decades. These perovskite-type materials are characterized by their high elastic modulus, their high dielectric constant, their low dielectric and elastic losses and high electro-mechanical coupling factors.

Although these piezoelectric ceramic materials have been successfully applied in a number of applications, they have a considerable number of limitations:
- Brittleness makes these materials prone to fracture and to the cracking propagation, which does not allow their use in applications in high-deformation sensors.
- The high density of ceramic materials, which creates problems in applications sensitive to the weight of the detector.
- The high acoustic impedance of ceramic materials (as a function of the density and of the rigidity).
- The pure starting materials and the basic processing costs of piezoelectric ceramics are relatively high per unit volume.

Many of these limitations can be overcome in specific applications using polymeric piezoelectric materials.

In 1969, Kawai et al. (Kawai, H., Jap. J. Appl. Phys., 8, 975, (1969)) developed a series of piezoelectric polymers which had strong piezoelectric activity in the temperature range between -40 and 90ºC, specifically poly(vinylidene fluoride) (PVDF) and its copolymers with trifluoroethylene and tetrafluoroethylene P(VDF/TrFE). In the 1980s, Scheinbeim et al. (Newman, B.A., Chen, P., Pae, K.D., and Scheinbeim, J.I., Journal Applied Physics, 51, 5161, (1980); and Scheinbeim, J.I., Journal Applied Physics, 52, 5939, (1981)) found an interesting piezoelectric activity in a series of semi-crystalline nylons, although they have not been marketed mainly as a result of the serious problem of moisture absorption. On the other hand, PVDF, which is currently one of the most known and marketed semi-crystalline piezoelectric polymers, loses its properties above 90ºC.

The literature on amorphous piezoelectric polymers is much more limited than for semi-crystalline systems. Piezoelectricity in amorphous polymers differs from that of semi-crystalline polymers and inorganic crystals in that polarization is not in a thermal equilibrium state, but rather in a quasi-stable state due to the molecular dipole freezing.

One of the most important properties of an amorphous piezoelectric polymer is its glass transition temperature (Tg), since it defines the use temperature and it defines the conditions of the polarization process. The orientation of the molecular dipoles by means of polarization is responsible for the piezoelectricity in amorphous polymers.

Thus, Broadhurst and Davis (Broadhurst, M.G., Harris, W.P., Mopsik, F.I., Malmberg, C.G., Polymer Preprint, 14, 820, (1973) established four criteria for considering that an amorphous or semi-crystalline polymer can show piezoelectric behavior.
i. There must be permanent dipoles in said polymer. These dipoles are generally pendant on the polymer backbone, though they can also be found within the main chain.
ii. The second criterion for piezoelectricity is the capability of the polymer to orient or align the molecular dipoles. This orientation is induced by applying an electric field (Ep) at a high temperature (Tp ≥ Tg) in which the chains are mobile enough, a dipolar alignment thus occurring. The partial retention of this orientation is achieved by reducing the temperature under the Tg while the applied electric field is maintained to freeze the polarized state. When this applied electric field is removed, certain remanent polarization will remain. This resulting remanent polarization (Pr) is directly proportional to the applied electric field and to the piezoelectric response (Scheinbeim, J.I., Journal Applied Physics, 52, 5939, (1981)).
iii. The third criterion for achieving a piezoelectric polymer is the capability for maintaining the dipolar alignment once it is reached.
iv. The final determining factor of the degree of piezoelectric response of a polymeric material is the capability of the polymer to be deformed with the applied mechanical stress.

Based on the fact that the remanent polarization in amorphous polymers is lost close to the Tg, the use of these piezoelectric polymers is limited to temperatures under the Tg.

Most works existing in the field of piezoelectric amorphous polymers focus on various polymers substituted with a nitrile group, such as polyacrylnitrile (PAN) (Newman, B.A., Chen, P., Pae, K.D., and Scheinbeim, J.I., Journal Applied Physics, 51, 5161, (1980); Scheinbeim, J.I., Journal Applied Physics, 52, 5939, (1981); Mathur, S.C., Scheinbeim, J.I., Newman, B.A. Journal Applied Physics, 56, 2419, (1984)); poly(vinylidene cyanide vinylacetate) (PVDCN/VAc) (Ueda, H., Carr, S.H., Polymer journal, 16, 661, (1984); Von Berlepsch, H., Pinnow, M., Stark, W.J. J. Phys. D.:Appl. Phys., 22, 1143, (1989); Von Berlepsch, H., Kunstler, W., Wedel, A.,Danz, R., Geiss, D. IEEE Trans. Elec. Ins., 24, 357, (1989); Jo, Y.S., Sakurai, M., Inoue, Y., Chujo, R., Tasaka, S., Miyata, S., Polymer, 28, 1583, (1987); and Miyata, S., Yoshikawa, M., Tasaka, S., Ko, M. Polymer journal, 12, 587, (1980)); polyphenylethernitrile (PPEN)(Furukawa, T., Tada, M., Nakajima, K. Seo, I. Jpn. J. Appl. Phys., 27, 200, (1988); Sakurai, M., Ohta, Y., Inoue, Y., Chujo, R., Polym. Comm., 32, 397, (1991)); and poly(1-bicyclo-butanecarbonitrile) (Tasaka, S., Inagaki, N., Okutani, T., Miyata, S., Polymer, 30, 1639, (1989)). The most promising materials are vinylidene cyanide copolymers, which show large dielectric relaxation stresses and strong piezoelectricity. Said compounds, however, have the drawbacks derived from their low thermal stability.

Recently, NASA Langley (Ounaies de, Z., Young, J.A., Harrison, J.S., NASA/TM-1999-209359; Park, C., Ounaies, Z., Wise, K.E., Harrison, J.S., NASA/CR-2002-211948; Park, C., Polymer, 45, 5417, (2004)) has synthesized polyimides containing a single cyano group in the repeating unit. They have specifically worked with the polyimides 2,4 (β-CN)APB/ODPA and 2,6 (β-CN)APB/ODPA prepared by means of reacting the diamines 2,4 or 2,6 (β-CN)APB (2,4- or 2,6-di(3-aminophenoxy)benzonitrile) and oxydiphthalic anhydride. Said polyimides, however, have the drawbacks of being too rigid and of having a low piezoelectricity.

NASA also proposes the use of polyimides in patent US 6,379,809, among other various polymeric substrates, with one or more cyano groups, among other polar groups, in the form of a film on which a metallic material is deposited, which assembly is subsequently polarized, thus providing a thermally stable material with piezoelectric and pyroelectric properties at temperatures greater than 100ºC and up to its Tg. However, said United States patent only illustrates the use of a polyimide with a cyano group and does not mention the preparation or the use of polyimides with more than one cyano group. The material obtained furthermore has the drawback of being rather inflexible.

Therefore there is still a need in the state of the art for novel materials having good piezoelectric properties at high temperatures, and which are flexible and lightweight.

The inventors have surprisingly found novel polyimides having a structural formula different from that of the aforementioned NASA compounds, which have two or three cyano groups in their structural unit and have good piezoelectric properties at a high temperature. Said polyimides have been able to be synthesized starting from novel diamines with 5 aromatic rings and with the cyano groups in alternating positions. The novel polyimides thus have a more flexible molecule and fewer steric hindrances, which allows better orientation of the dipoles in the applied electric field direction and, therefore, better piezoelectricity values. Said polyimides until now have not been synthesized due to the difficulty of obtaining the monomeric material.

These novel synthesized polyimides are furthermore stable at high temperatures and have the additional advantages of being flexible and lightweight, therefore the range of applications is very broad in the industry field.

### Object of the Invention

The object of the present invention is therefore to provide polyimides with good piezoelectric properties at high temperatures.

Another object of the invention is to provide a process for the preparation of said polyimides.

Another object of the invention is to provide diamines for the preparation of said polyimides.

Likewise, another object of the invention is to provide a process for the preparation of said diamines.

Finally, another object of the invention is to provide the use of said polyimides in industrial applications.

### Description of the Drawings

Figure 1 shows the DSC (differential scanning calorimetry) curve obtained for the polyimide of formula (Ia) without polarizing. The energy variation of the sample is depicted on the Y-axis and the temperature on the X-axis.
Figure 2 shows the TG (thermogravimetry) curve of the polyimide of formula (Ia) without polarizing. The mass variation of the sample is depicted on the Y-axis and the temperature on the X-axis.
Figure 3 shows the TSDC (thermally stimulated depolarization current) curve of the polyimide of formula (Ia). The depolarization current intensity variation of the sample is depicted on the Y-axis and the temperature on the X-axis.
Figure 4 shows the remanent polarization variation with the temperature of the polyimide of formula (Ia).
Figure 5 shows the diagram of the method for the polarization of the polyimide of formula (Ia).

### Detailed Description of the Invention

The present invention provides a polyimide with piezoelectric properties at high temperatures having a repeating structural unit of formula (I): 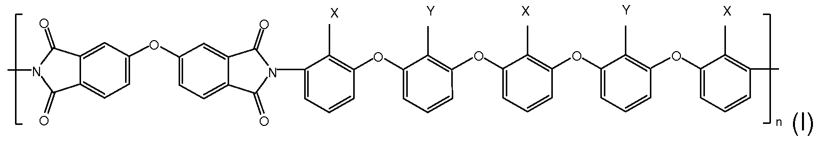 wherein X and Y represent hydrogen and cyano, with the proviso that when one of X and Y is hydrogen, the other one is cyano, hereinafter "polyimide of the invention".

In the context of the invention, the term "polyimide with piezoelectric properties at high temperatures" relates to a polyimide having piezoelectric properties at temperatures greater than 90ºC.

In a particular embodiment, the polyimide of the invention has a repeating structural unit of formula (Ia):

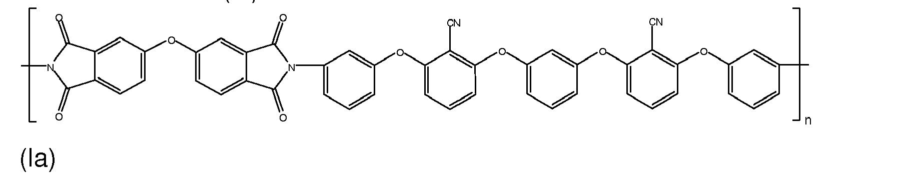

In another particular embodiment, the polyimide of the invention has a repeating structural unit of formula (Ib):

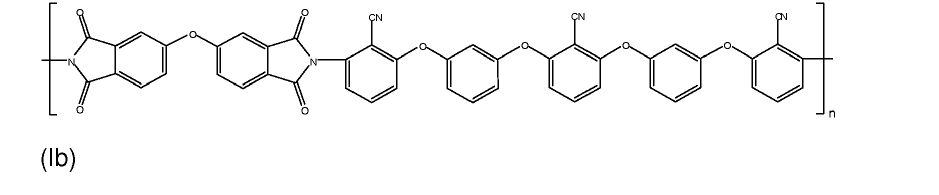

In another aspect of the invention, a process is provided for the preparation of the previously described polyimide with piezoelectric properties at high temperatures, hereinafter "process of the invention", comprising the steps of:
(a) reacting a diamine of structural formula (II): 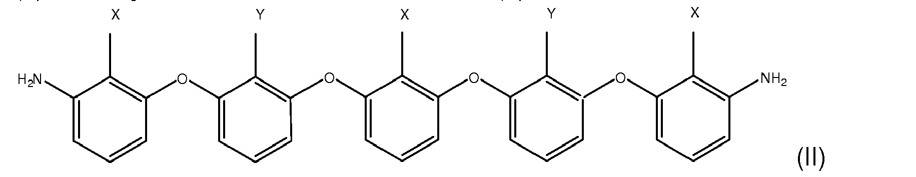 wherein X and Y have the previously mentioned meanings for the polyimide with the repeating structural unit of formula (I);
   with 4,4'-oxydiphthalic anhydride to obtain a polyamic acid of formula (III): 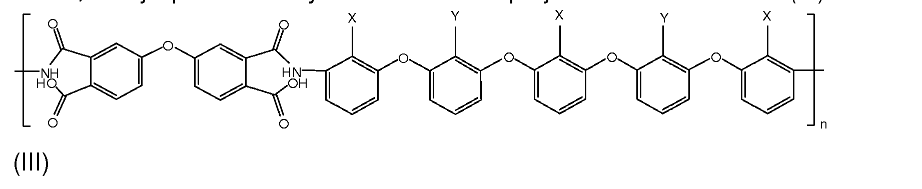 wherein X and Y have the previously mentioned meanings;
(b) curing said polyamic acid of formula (III) by means of thermal treatment to obtain the polyimide of formula (I): 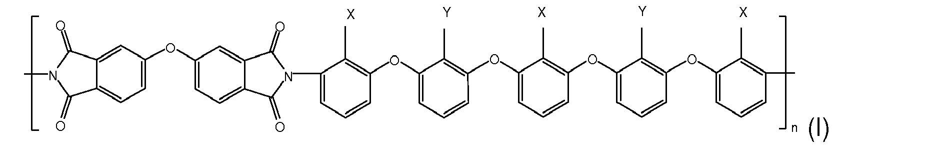 wherein X and Y have the previously mentioned meanings; and
(c) polarizing said polyimide of formula (I) by means of thermal treatment and simultaneous application of an electric field.

Commercial 4,4'-oxydiphthalic anhydride is used in step (a). Likewise, any suitable inert solvent of the state of the art can be used as a solvent, such as, for example, N,N-dimethylacetamide (DMAc), dimethyl sulfoxide (DMSO), N,N-dimethylformamide (DMF) or N-methylpyrrolidone (NMP), preferably N,N-dimethylacetamide. In relation to the reaction conditions of step (a), the reaction of the corresponding diamine with the 4,4'-oxydiphthalic anhydride is carried out in an inert atmosphere (nitrogen atmosphere, for example) and under stirring, at a temperature of -15 to 100ºC, preferably at room temperature, and for a time of 4 to 30 hours, preferably 24 hours.

Once the corresponding polyamic acid is obtained, its curing is performed for the purpose of causing the cyclization thereof. For this purpose, it is subjected to heating at a temperature greater than 100ºC, of the order of 20ºC greater than its glass transition temperature. This can be estimated in an approximate manner by performing a preliminary differential scanning calorimetry or DSC analysis with several milligrams of the product obtained in step (a), as indicated below.

Therefore, in a particular embodiment of the process of the invention, the curing of step (b) is performed by means of heating at a temperature of 180-220ºC. In a preferred embodiment, said curing is performed by means of heating at a temperature of 200ºC. Said heating of step (b) can be performed by conventional means, such as, for example, using a muffle. Likewise, the heating cycle until reaching the desired temperature will be determined by the person skilled in the art, subjecting the polyamic acid to temperature ramps and isotherms at a slow enough rate so that the solvent has the time necessary to evaporate without bubbling.

In its natural state, the polyimide obtained after step (b), it has no pyroelectric or piezoelectric properties because its dipoles are randomly oriented. To achieve this orientation, they are polarized by means of applying a high intensity electric field, simultaneously heating at a temperature greater than its glass transition temperature.

Therefore, in a particular embodiment of the process of the invention, the polarization is performed by means of heating at a temperature of 180-210ºC and simultaneous application of an electric field of 5-9 kV. In a preferred embodiment, the polarization is performed by means of heating at a temperature of 200ºC and simultaneous application of an electric field of 7.5 kV.

For that purpose any polarization method of the state of the art can be used, such as the corona polarization method, for example, which consists of using a silver plated face of the sample as one electrode, whereas the second electrode is a tungsten tip located at 1 cm from the non-silver plated face such that it allows maintaining the sample in the intended constant intensity electrostatic field. Once the polarization of the polyimide has ended, it is left to cool at room temperature maintaining the applied electric field.

In another aspect of the invention, a diamine of structural formula (II) used in step (a) of the previously described process of the invention is provided: 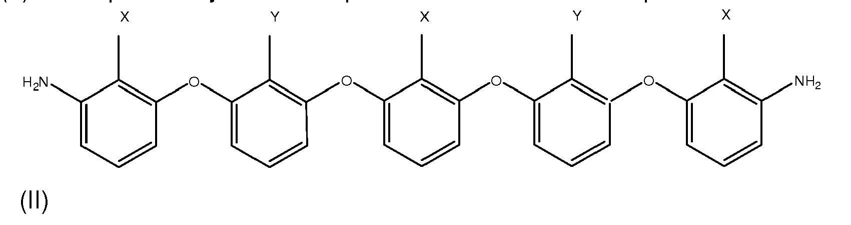 wherein X and Y have the previously mentioned meanings for formula (I).

In a preferred embodiment, said diamine has structural formula (IIa):

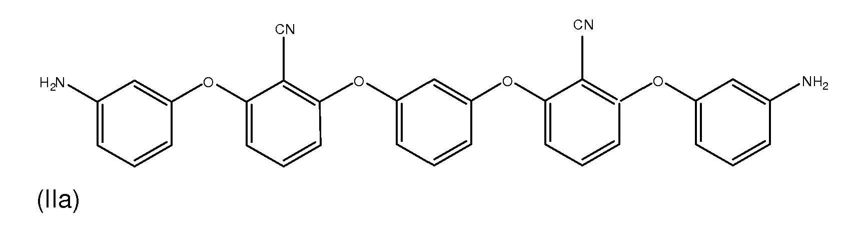

In another preferred embodiment, said diamine has structural formula (IIb):

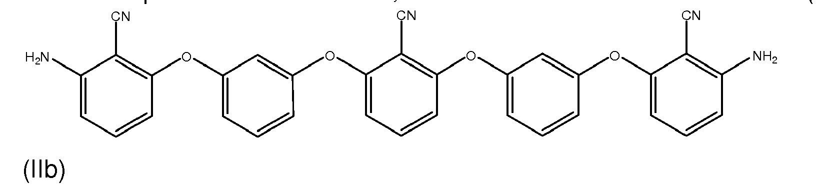

In another aspect of the invention, a process for preparing the diamine of formula (II) is provided comprising the steps of:
(i) reacting two equivalents of the compound of formula (IV): 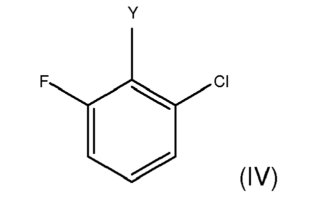 wherein Y represents hydrogen or cyano; with an equivalent of the compound of formula (V): 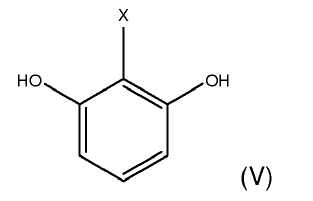 wherein X represents hydrogen or cyano, with the proviso that when one of X and Y is hydrogen, the other one is cyano, to obtain the compound of formula (VI): 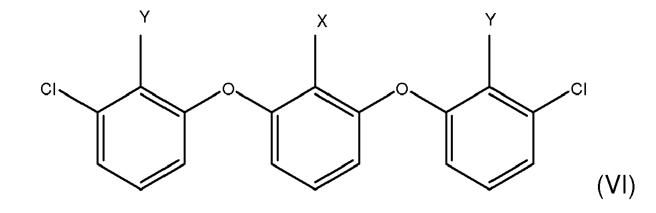 wherein X and Y represent hydrogen or cyano, with the proviso that when one of X and Y is hydrogen, the other one is cyano; and
(ii) reacting an equivalent of the compound of formula (VI) obtained with two equivalents of the compound of formula (VII) 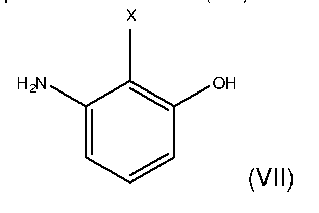 wherein X represents hydrogen or cyano, with the proviso that when one of X and Y is hydrogen, the other one is cyano.

A suitable inert solvent is used in step (i) such as, for example, N,N-dimethylacetamide (DMAc), dimethyl sulfoxide (DMSO), N,N-dimethylformamide (DMF) or N-methylpyrrolidone (NMP), preferably N,N-dimethylacetamide. With regard to the reaction conditions of step (i), the reaction of dihalogenated benzene of formula (IV) with the resorcinol derivative of formula (V) is carried out in the presence of a suitable base (potassium carbonate or sodium carbonate, for example), under continuous stirring, at a temperature of 130 to 170ºC, preferably at 150ºC, and for a time of 12 to 48 hours, preferably 17 hours.

On the other hand, any suitable inert solvent of the state of the art is used as a solvent in step (ii) such as, for example, dimethyl sulfoxide (DMSO), N,N-dimethylacetamide (DMAc), N,N-dimethylformamide (DMF) or N-methylpyrrolidone (NMP), preferably dimethyl sulfoxide. In relation to the reaction conditions of step (ii), the reaction of the premonomer of formula (VI) with the aminophenol of formula (VII) is carried out in the presence of a suitable base (potassium carbonate or sodium carbonate, for example), in an inert atmosphere (nitrogen atmosphere, for example) and under stirring, at a temperature of 110 to 150ºC, preferably at 130ºC, and for a time of 24 to 48 hours, preferably 40 hours.

In another aspect of the invention, the use of the previously described polyimide with piezoelectric properties at high temperatures in industrial applications is provided. In a preferred embodiment, the polyimide of the invention is used to manufacture sensors, actuators, microelectromechanical devices, pyroelectric infrared radiation detectors and devices for aerospace applications.

The following examples illustrate the invention and must not be considered as limiting of the scope thereof.

### EXAMPLE 1

### Preparation of the polyimide of formula (Ia)

### 1.1.- Preparation of the 1,3-bis-[2-cyano-3-(3-aminophenoxy)-phenoxy]benzene (diamine of formula (IIa)).

9.85 g of 2-chloro-6-fluorobenzonitrile were mixed with 3.48 g of resorcinol at a 2:1 ratio and a K₂CO₃ excess was added. N,N-dimethylacetamide (DMAc) was used as solvent and the reaction was maintained at 150ºC for 17 hours in a nitrogen atmosphere and under continuous stirring.

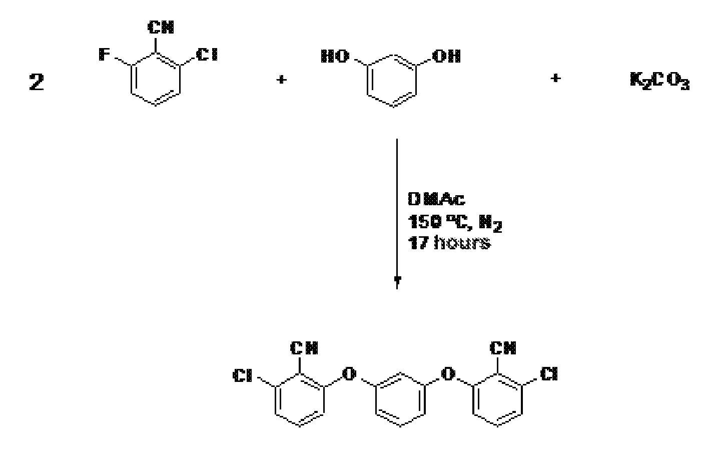

After that time has elapsed, the reaction mixture was tempered and a solution of dichloromethane/Millipore water was added. Both phases were separated with the aid of a separating funnel, the premonomer being dissolved in the organic phase. Then, the organic phase was dried with MgSO₄. Finally, the solvent was evaporated under reduced pressure, 1,3-bis(2-cyano-3-chlorophenoxy)benzene thus being obtained with an 87% yield. Finally, the compound was characterized, determining that it was the expected substance.

### NMR

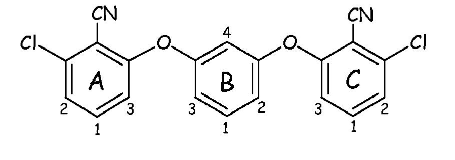

¹H-NMR (500 MHz, CDCl₃): δ (ppm) = 7.49, 7.46 (dt, J = 8.2, Hz, J = 8.4 Hz, 3H, A1, B1, C1); 7.26 (d, J = 8.2 Hz, 2H, A2, C2); 6.99 (dd, J = 8.2, J = 2.2 Hz, 2H, B2, B3); 6.88 (d, J = 8.4 Hz, 2H, A3, C3); 6.85 (t, J = 2.2 Hz, 1H, B4).

¹³C-NMR (125.7 MHz, CDCl₃): δ (ppm) = 160.2 (Cₐᵣ-OPh, A, C rings); 156.1 (Cₐᵣ-OPh, B ring); 138.3 (Cₐᵣ-Cl, A, C); 134.3 (t-Cₐᵣ, A1, C1); 131.5 (t-Cₐᵣ, B1); 124.3 (t-Cₐᵣ, A2, C2); 116.6 (t-Cₐᵣ, B2, B3); 115.4 (t-Cₐᵣ, A3, C3); 112.9 (CN); 111.7 (t-Cₐᵣ, B4); 105.4 (Cₐᵣ-CN).

IR (KBr): γ (cm⁻¹) = 3116-2984; 2231; 1636-1437; 1253; 1033; 853-793.

Elemental analysis obtained for C₂₀H₁₀N₂O₂Cl₂: C, 62.99%, H, 2.62%, N, 7.35%. Found: C, 64.62%, H, 2.88%, N, 7.73%.

Then, 10 g of prepared 1,3-bis(2-cyano-3-chlorophenoxy)benzene were reacted with 5.72 g of 3-aminophenol at a 1:2 ratio and a K₂CO₃ excess was added. Dimethyl sulfoxide (DMSO) was used as solvent and the reaction was maintained at 130ºC for approximately 40 hours in an inert atmosphere (N₂) and under continuous stirring.

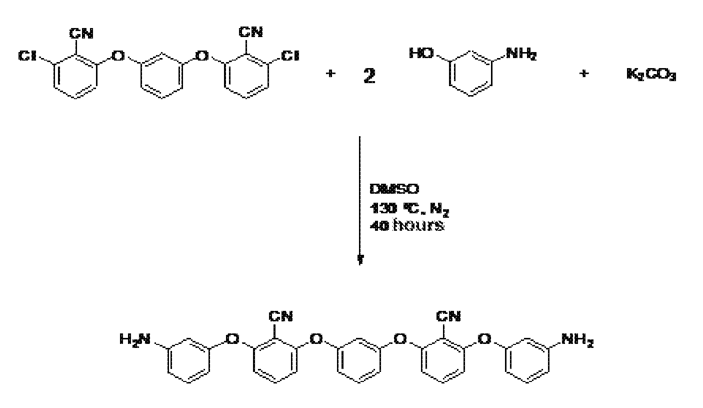

After the reaction time, it was precipitated with Millipore water, centrifuged at 4000 rpm for 40 minutes and was decanted. The decanted material was recrystallized recrystallized from a mixture of EtOH/H₂O (1:1). It was again separated by decanting and was dried under reduced pressure, the reaction yield being 74%. Finally, said diamine was characterized by different techniques and it was verified that the desired structure was obtained.

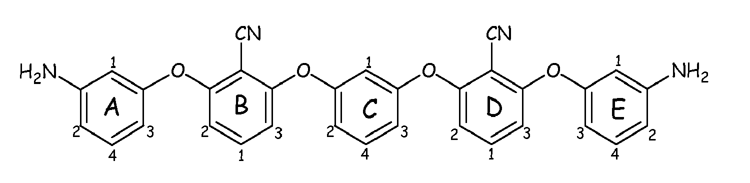

¹H-NMR (500 MHz, DMSO-d6]: δ (ppm) = 7.57-7.51 (m, 3H, B1, D1, C4); 7.18-7.16 (m, 1H, C1); 7.12 (dd, J = 8.2 Hz, J = 2.1 Hz, 2H, C2, C3); 7.08 (t, J = 8.0 Hz, 2H, A4, E4); 6.80 (d, J = 8.5 Hz, 1H, B2/B3/D2/D3); 6.72 (d, J = 8.4 Hz, 1H, B2/B3/D2/D3); 6.65 (d, J = 8.5 Hz, 1H, B2/B3/D2/D3); 6.59 (d, J = 8.4 Hz, 1H, B2/B3/D2/D3); 6.47 (dd, J = 8.0 Hz, J = 1 Hz, 2H, A3/E3); 6.33-6.32 (m, 2H, A1/E1); 6.27 (dd, J = 7.9 Hz, J = 1.7 Hz, 2H, A2/E2); 5.38 (s, 4H, NH₂).

¹³C-NMR (125.7 MHz, DMSO-d6): δ (ppm) = 161.2, 160.2, 156.1, 151.4 (quaternary Cₐᵣ); 136.3, 132.4, 130.9 (meta Cₐᵣ with respect to substituents); 116.6 (CN); 113.6, 112.3, 111.9, 111.4, 110.5, 106.5 (ortho Cₐᵣ with respect to substituents); 95.1 (Cₐᵣ-CN).

IR (KBr): γ (cm⁻¹) =3452 -3222; 3078; 2223; 1587-1455; 1244; 1030; 854-677.

Elemental analysis obtained for C₃₂H₂₂N₄O₄: C, 73.00%, H, 4.18%, N, 10.65%. Found: C, 67.10%, H, 4.91 %, N, 9.92%.

### 1.2.- Preparation of polyamic acid.

5.94 g of commercial 4,4'-oxydiphthalic anhydride (Merck, 97%) were equimolecularly mixed with 10.08 g of the diamine prepared in step 1.1 using N,N-dimethylacetamide (DMAc) as solvent and adjusting the solid content to 20%. Both substances were dissolved separately and once dissolved, the dianhydride was poured on the reaction flask in which the diamine was located. The reaction was carried out in a nitrogen atmosphere, at room temperature and under continuous stirring.

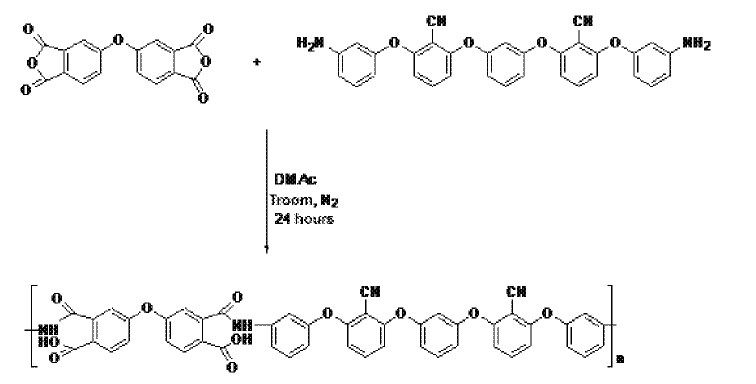

After approximately 24 hours of reaction, it was washed several times with cold MeOH, was decanted and the solid was dried under reduced pressure, the yield being 48%. Next, the polyamic acid obtained was characterized, concluding that the desired structure had been obtained.

¹H-NMR (500 MHz, DMSO-d6): δ (ppm) = 13.15 (bs, 2H, OH); 10.59 (s, 2H, NH).

IR (KBr): γ (cm⁻¹) = 3261 (OH); 3189 (secondary NH); 3074; 2231; 1719-1605; 1491-1461; 1245; 1028; 967-678.

Elemental analysis obtained for C₄₈H₂₄N₄O₉ (completely cured): C, 72.00%, H, 3.00%, N, 7.00%. Found: C, 71.59%, H, 2.94%, N, 7.18%.

The values of each of the atoms were determined in the elemental analysis for completely cured samples due to the fact that the polyamic acid contained a lot of occluded solvent within the material and falsified the results. Therefore, it was previously heated to 300ºC and then the measurement was taken.

Prior to preparing the polyimide of formula (Ia), the glass transition temperature and the thermal stability thereof were determined from a sample of the polyamic acid obtained.

### I. Determination of the glass transition temperature.

When the properties of the amorphous polymers are studied as a function of temperature, a temperature or rather a relatively narrow region of temperatures is observed in which a strong change in the physical and mechanical properties is presented: above this region of temperatures the polymer is soft and acts like a viscous liquid whereas under it the polymer is hard, rigid and brittle, being the temperature separating these two behaviors the glass transition temperature or Tg.

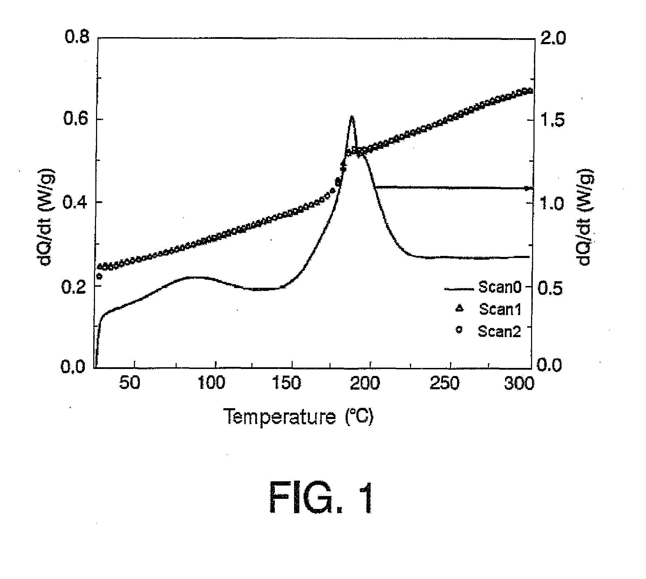

Said parameter was determined by means of the differential scanning calorimetry or DSC technique using a METTLER TOLEDO DSC 822e calorimeter. For that purpose, a sample of approximately 10 mg of the polyamic acid obtained in step 1.2 was subjected to heating from room temperature to 300ºC at a rate of 20ºC/min and the energy changes which the sample experienced with the temperature were measured, i.e., the heat that had to be supplied to a sample or removed from it for maintaining its temperature equal to that of a reference was measured. The results are shown in Figure 1, wherein the energy variation which the polyimide of formula (Ia) experiments without polarizing as a function of temperature is represented.

Several scans were performed until obtaining reproducible thermograms. Therefore, two very sharp endothermic peaks were observed in the first scan (scan0) due to curing or cyclization of the polyamic acid and to the evaporation of the residual solvent remaining occluded therein, respectively. In the second and third scans (scan01 and scan02), the variation was virtually constant. From said curves, the Tg of the polyimide was determined as the intersection between the extrapolation of the baseline and the tangent to the curve in the inflection area, a value of 176-3ºC being obtained.

### II. Determination of the thermal stability

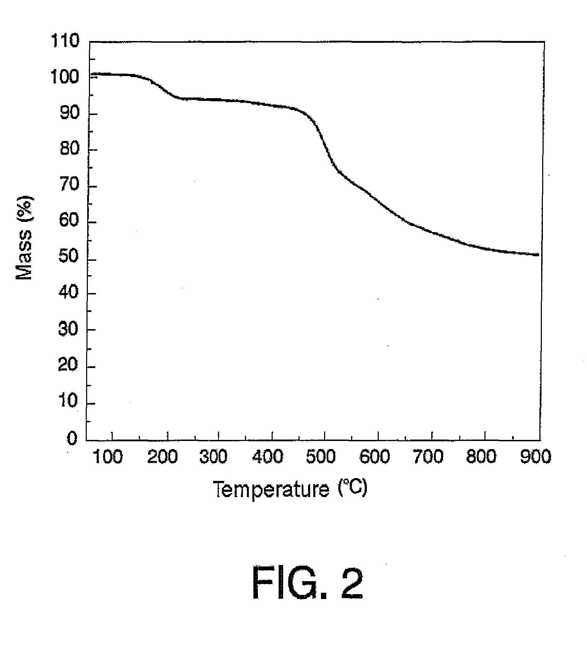

The thermogravimetry or TG technique was used using a METTLER TOLEDO TGA/SDTA 851 e thermoscale. Therefore, a sample of approximately 10 mg of the polyamic acid obtained in step 1.2 was subjected to heating from 50 to 900ºC at a rate of 10ºC/min and the mass loss thereof was measured. The results are shown in Figure 2, in which the mass variation of the polyimide of formula (Ia) without polarizing as a function of temperature is shown.

The first step observed (area of lower slope) is a consequence of the curing of the polyamic acid and of the evaporation of the residual solvent; whereas the second step (area of greater slope) is a consequence of the degradation of the sample. It can be concluded that the polyimide is very thermally stable since the onset of degradation takes place around 450ºC, approximately half the mass remaining as a carbonaceous residue.

### 1.3.- Curing of the polyamic acid to obtain poly[(1,3-dihydro-1,3-dioxo-2H-isoindole-2,5-diyl)oxy(1,3-dihydro-1,3-dioxo-2H-isoindole-5,2-diyl)-1,4-oxyphenylene-oxy(2-cyano-1,3-phenylene)-1,3-oxyphenylene-oxy(2-cyano-1,3-phenylene)-1,3-phenylene (polyimide of formula (Ia))

The polyamic acid obtained in step 1.2 was introduced in a muffle and was heated at 200ºC (calculated Tg + approx. 20ºC), subjecting it to the following heating cycles:
- it was heated from room temperature to 150ºC for 48 hours;
- it was maintained at 150ºC for 2 hours;
- it was heated from 150ºC to 200ºC for 35 hours; and
- it was maintained at 200ºC for 2 hours.

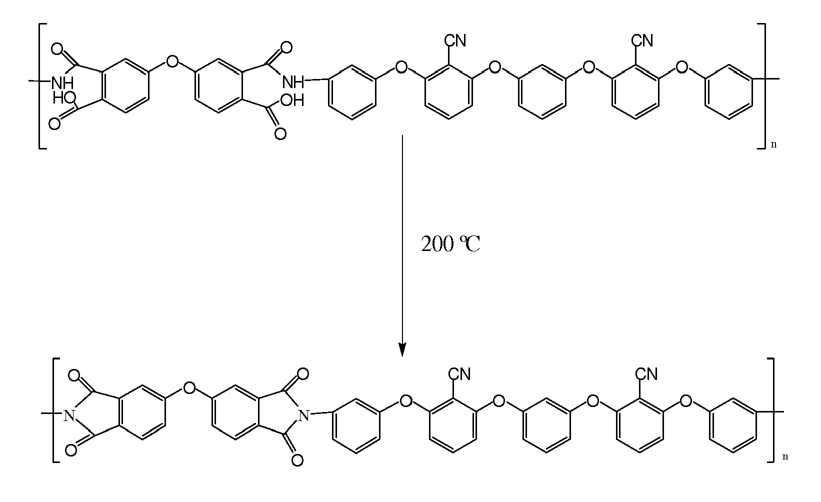

Finally, the cured polyimide was allowed to cool in the muffle to room temperature before taking it out.

### 1.4.- Polarization of poly[(1,3-dihydro-1,3-dioxo-2H-isoindole-2,5-diyl)oxy(1,3-dihydro-1,3-dioxo-2H-isoindole-5,2-diyl)-1,4-oxyphenylene-oxy(2-cyano-1,3-phenylene)-1,3-oxyphenylene-oxy(2-cyano-1,3-phenylene)-1,3-phenylene (polyimide of formula (Ia))

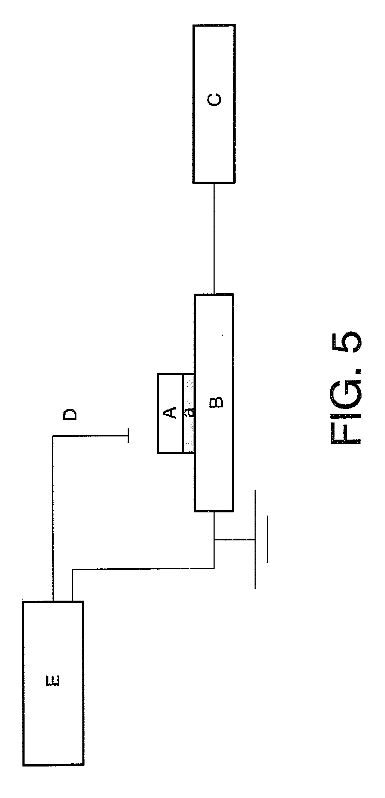

The polyimide synthesized in step 1.3 had an amorphous structure with its dipoles randomly oriented. In order for it to acquire piezoelectric properties it was necessary to polarize it. For that purpose, a high voltage electric field was applied at a temperature above the Tg. The dipoles thus acquired sufficient mobility to be able to be oriented in the applied field direction. The diagram of the polarization method is shown in Figure 5.

The polymeric sample (A) with a silver plated face (a) was placed on a copper block (B) assuring at all times thermal and electric contact between both parts. The temperature of the block (B) was controlled with a temperature regulator (C). A tungsten tip (D) was placed at a distance of 1 cm from the non-silver plated face of the sample and a high voltage field (7.5 kV) was applied using a HITEK Power 4000 Series power source (E).

The sample of polyimide was heated at a rate of 2ºC/min to a temperature of 200ºC (±2ºC) with an applied electric field. Once this temperature was reached, it was maintained at the same temperature for approximately 1 hour and, then with an applied field, the temperature was gradually reduced at a rate of 5ºC/min to room temperature.

### EXAMPLE 2

### Physical characterization of the polyimide of formula (Ia).

### Determination of the remanent polarization

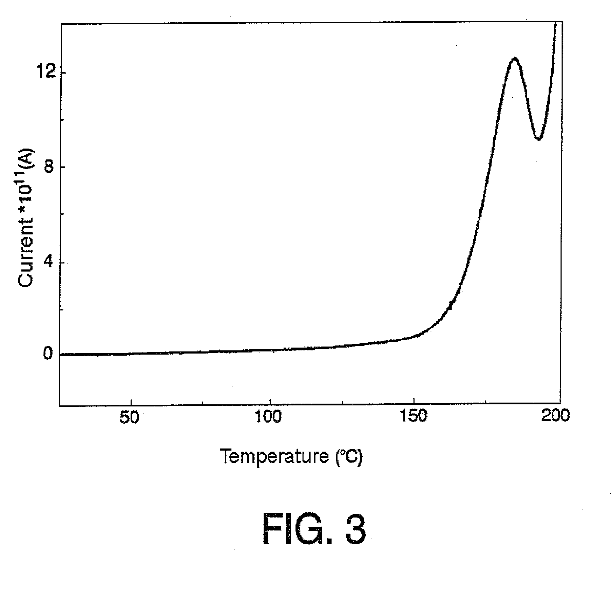

The remanent polarization value, dependent on the dipolar moment value of the polymer and on the degree of orientation of the dipoles in the applied field direction, was measured using the Thermally Stimulated Depolarization Current (TSDC) method (Physical Review B, 50, (17), 12489, (1994)). The sample was placed in a cryostat (THMSE 600, LINKAM) which allowed changing its temperature from room temperature to 200ºC at a constant rate (1ºC/min). During the heating process, the remanent polarization value of the sample changed. The electric current produced during this depolarization process of the polymer was measured using a KEITHLEY 6514 electrometer. The data acquisition (current values as a function of temperature and time) as well as the measurement conditions were controlled with a PC. Figure 3 shows a current variation as a function of temperature.

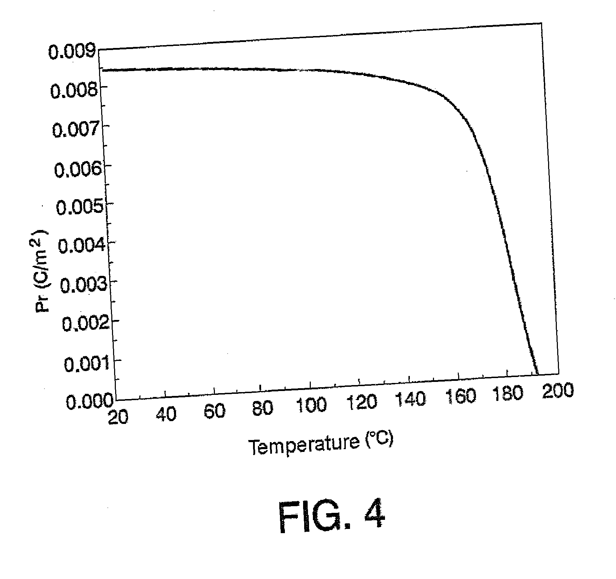

Representing the curve of Figure 3 as a function of time, integrating and knowing the dimensions of the sample, the dependence of the remanent polarization (Pr) is calculated as a function of temperature. This relationship is shown in Figure 4.

The Pr value at 20ºC for the polyimide of formula (Ia) of the invention is of 8.43 mC/m². As can be observed in Figure 4, the Pr value is maintained virtually constant until temperatures close to 150ºC (the relative change of the polarization between these temperatures is less than 10%) which thus assures constant piezoelectric coefficient values. This makes the polyimide of formula (Ia) of the invention very interesting for applications at a high temperature in piezo- and pyroelectric detectors.

As previously indicated, PVDF is one of the most widely known and most marketed polymers for applications in piezo- and piezoelectric detectors. However, this can only be used up to the temperature of 90ºC.

In previous investigations for polyimides with a single cyano group (Park et al; 2004, mentioned above) synthesized and cured in the same manner as the polyimide (Ia) of the invention, Pr values of 3 mC/m² are obtained at 20ºC, which involves a 280% increase for the polyimide of the invention.

## Claims

1. Polyimide with piezoelectric properties at high temperatures **characterized in that** it has a repeating structural unit of formula (I): 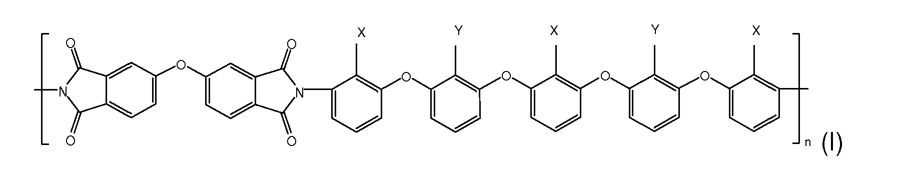 wherein X and Y represent hydrogen and cyano, with the proviso that when one of X and Y is hydrogen, the other one is cyano.

2. Polyimide according to claim 1, **characterized in that** it has a repeating structural unit of formula (Ia):

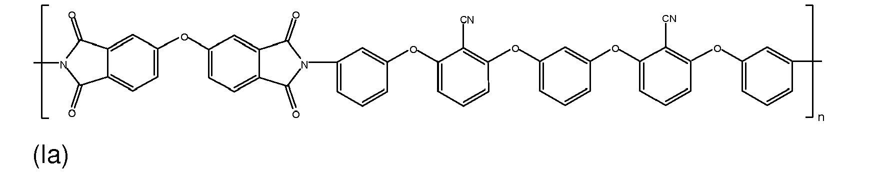

3. Polyimide according to claim 1, **characterized in that** it has a repeating structural unit of formula (Ib):

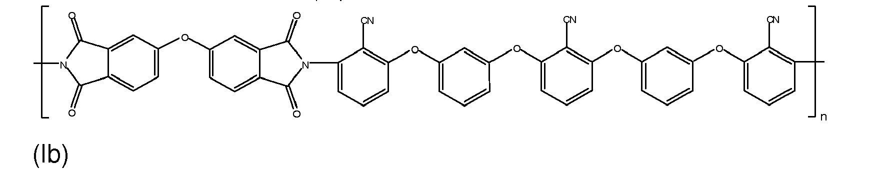

4. Process for the preparation of a polyimide with piezoelectric properties at high temperatures according to claims 1-3, **characterized in that** it comprises the steps of:
(a) reacting a diamine of structural formula (II): 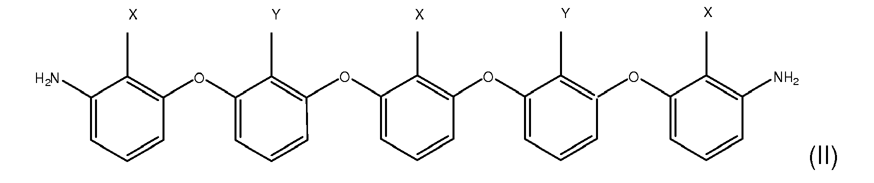 wherein X and Y have the meanings given in claim 1;
with 4,4'-oxydiphthalic anhydride to obtain a polyamic acid of formula (III): 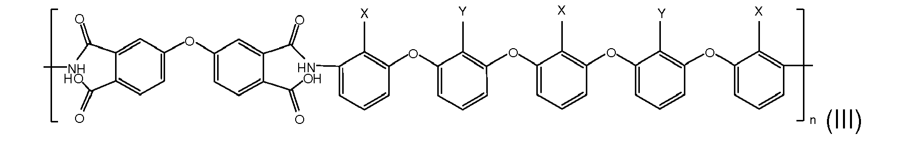 wherein X and Y have the meanings given in claim 1;
(b) curing said polyamic acid of formula (III) by means of thermal treatment to obtain the polyimide of formula (I): 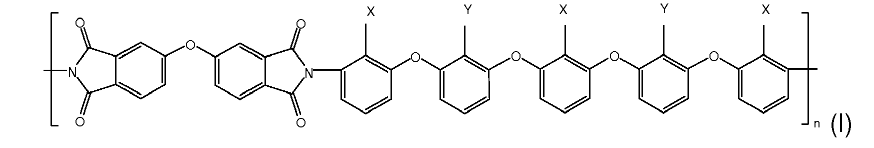 wherein X and Y have the meanings given in claim 1; and
(c) polarizing said polyimide of formula (I) by means of thermal treatment and simultaneous application of an electric field.

5. Process according to claim 4, **characterized in that** in step (b) the curing is performed by means of heating at a temperature of 180-220ºC.

6. Process according to claim 5, **characterized in that** in step (b) the curing is performed by means of heating at a temperature of 200ºC.

7. Process according to claim 4, **characterized in that** in step (c) the polarization is performed by means of heating at a temperature of 180-210ºC and simultaneous application of an electric field of 5-9 kV.

8. Process according to claim 7, **characterized in that** in step (c) the polarization is performed by means of heating at a temperature of 200ºC and simultaneous application of an electric field of 7,5 kV.

9. Diamine used in the process of claims 4-8, **characterized in that** it has structural formula (II): 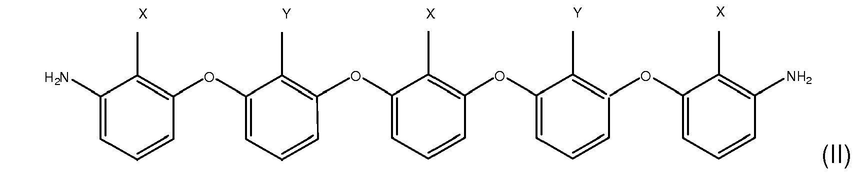 wherein X and Y have the meanings given in claim 1.

10. Diamine according to claim 9, **characterized in that** it has structural formula (IIa):

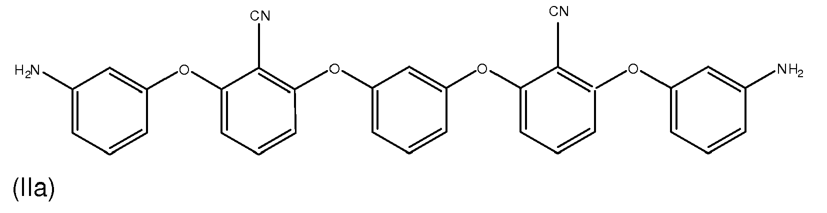

11. Diamine according to claim 9, **characterized in that** it has structural formula (IIb):

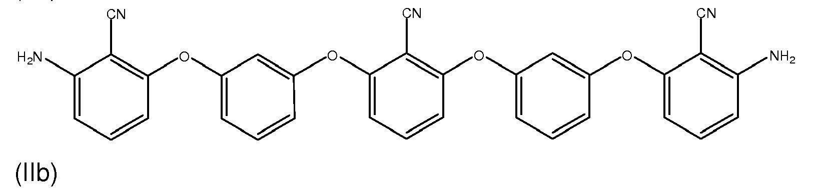

12. Process for the preparation of the diamine of formula (II) according to claims 9-11, **characterized in that** it comprises the steps of:
(i) reacting two equivalents of the compound of formula (IV): 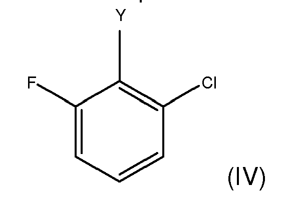 wherein Y represents hydrogen or cyano; with an equivalent of the compound of formula (V): 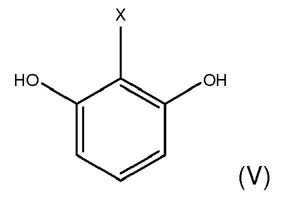 wherein X represents hydrogen or cyano, with the proviso that when one of X and Y is hydrogen, the other one is cyano, to obtain the compound of formula (VI): 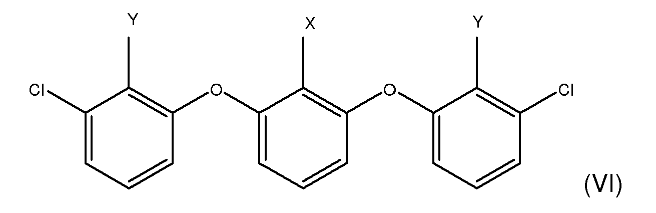 wherein X and Y represent hydrogen or cyano, with the proviso that when one of X and Y is hydrogen, the other one is cyano; and
(ii) reacting an equivalent of the compound of formula (VI) obtained with two equivalents of the compound of formula (VII) 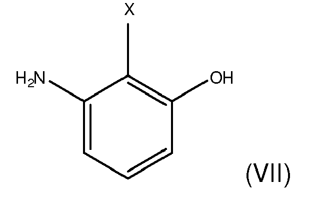 wherein X represents hydrogen or cyano, with the proviso that when one of X and Y is hydrogen, the other one is cyano.

13. Use of the polyimide with piezoelectric properties at high temperatures according to claims 1-3 in industrial applications.

14. Use according to claim 13 for the manufacture of sensors, actuators, microelectromechanical devices, pyroelectric infrared radiation detectors and devices for aerospace applications.
